# EUROPEAN PATENT SPECIFICATION

(11) **EP 3 209 279 B1**
(45) Date of publication and mention of the grant of the patent: **24.04.2019**
(21) Application number: 15771426.2
(22) Date of filing: 17.09.2015
(51) Int. Cl.: A61K 8/67, A61Q 19/00, A61Q 19/08

(54) **SKIN CARE PRODUCTS**
HAUTPFLEGEPRODUKTE
PRODUITS DE SOINS DE PEAU

(30) Priority: 17.09.2014 US 201462051770 P
(43) Date of publication of application: 30.08.2017
(73) Proprietor: The Procter & Gamble Company, Cincinnati, OH 45202 (US)
(72) Inventor: MCILDOWIE, Matthew James, Leedervilee, Western Australia 6007 (AU); EDWARDS, Jeffrey, David, Leedervilee, Western Australia 6007 (AU)
(74) Representative: Engisch, Gautier
(86) International application number: PCT/US2015/050645
(87) International publication number: WO 2016/044567

(56) References cited:
- WO-A1-2011/146977
- WO-A1-2011/146978
- WO-A1-2011/156869
- WO-A1-2012/031335
- US-A1- 2006 222 689
- US-A1- 2007 053 858

## Description

### FIELD OF THE INVENTION

The present invention relates to skin care products that provide enhanced penetration of a skin care active into skin, and to a method for enhancing delivery of a skin care active into skin. Specifically, the present invention relates to the pairing of an applicator and a skin care composition including skin care actives that have particular diamagnetic properties.

### BACKGROUND OF THE INVENTION

Topical skin care compositions containing actives that provide benefits to skin are well known. For example, Vitamin B3 compounds, particularly niacinamide are known to provide measurable skin regulating benefits. Topical niacinamide is known to help regulate the signs of skin aging, by reducing the visibility of the fine lines, wrinkles, and other forms of uneven or rough surface texture associated with aged or photo-damaged skin. These compounds have also been found useful in reducing the overall oiliness of skin.

However, effective and optimal delivery of skin care actives, such as niacinamide, into skin is an ongoing challenge. Typically, active agents with skin care benefits are introduced to skin via topical application of, for example, creams, lotions and essences. However, the actual and perceived benefits of skin care actives such as niacinamide are largely dependent on the amount of skin care active that penetrates the top layer of skin and the depth to which it penetrates. There are various factors that limit the amount of active agent that can penetrate skin, and at present there is little control over the positioning and residency of the active agents following penetration into skin.

The amount of active agent provided in a skin care composition can be increased in various ways, for example, by increasing the amount of active agent in the skin care composition. However, this often leads to compositions that do not have a good sensory feel, increased formulation challenges, stability issues and increased manufacturing costs.

One approach to improving the efficacy of a skin care active is to use chemical penetration enhancers to facilitate changes in skin permeability, allowing enhanced penetration of the skin care active. However, the use of chemical penetration enhancers can be problematic due to unknown interaction with the active agent and the potential for adverse side effects such as irritation of skin and mucosal surfaces.

Mechanical approaches to increasing skin penetration of actives have also been explored. For example, one such approach known as iontophoresis utilizes an electrical energy gradient to accelerate a charged active agent(s) across the skin (or other barrier). An example of a device that uses iontophoresis is described in US 7,137,965. However, iontophoresis is only suitable for specific active agents with certain ionic structures and can be injurious to certain dermal barriers due to exchange ion degradation. Additionally, iontophoresis requires the use of intimate electrical contact and adhesive electrodes, which are not suitable for all target surfaces or barriers.

Other techniques for creating mobility and/or direction in the movement of active agent(s) include magneto kinetics and magneto-phoresis. However, these techniques have been difficult to implement due to poor performance, high hardware and energy requirements, and cost. An example of a device that utilizes magnetophoresis is described in US 2009/0093669. While these methods claim to increase the amount of penetration of skin care actives into skin, they still do not provide enhanced penetration in a controlled manner - both in terms of amount of penetration and depth of penetration.

In another example of a device designed to effectively deliver skin care actives, WO 2011/156869 discloses a method of delivering a skin care agent through a dermal barrier using one or more displaced dipolar magnetic elements. However, this method still does not provide a targeted approach that takes account of the unique properties and targeted benefit areas in skin of different skin care actives.

Accordingly, there is a need to provide a skin care product that can provide improved penetration of specific skin care actives into skin in a controlled manner.

### SUMMARY OF THE INVENTION

According to a first aspect of the invention, there is provided a cosmetic skin care product, comprising an applicator comprising a magnetic array, the magnetic array having a first layer of one or more dipolar pairs of alternating magnetic poles with a first layer pitch of between 1mm and 3.5mm, and a first layer magnetic field strength of between 12mT and 30mT; and a skin care composition comprising an effective amount of a vitamin B3 active and a dermatologically acceptable carrier.

According to a second aspect of the invention, there is provided a method of making a skin care product, comprising the steps of: providing a skin care composition, the skin care composition including a vitamin B3 active; constructing a magnetic array for use with the skin care composition by magnetizing a ferromagnetic substrate with at least a first layer of one or more dipolar pairs of alternating magnetic poles with a first layer pitch of between 1mm and 3.5mm, and a first layer magnetic field strength of between 12mT and 30mT; joining the magnetic array to an applicator.

According to a third aspect of the invention, there is provided a method of cosmetically regulating a skin condition, comprising the steps of: applying a skin care composition to a target portion of skin, the skin care composition including a vitamin B3 active; positioning an applicator having a magnetic array embedded therein above the target portion of skin, the magnetic array comprising at least a first layer of one or more dipolar pairs of alternating magnetic poles with a first layer pitch of between 1mm and 3.5mm, and a first layer magnetic field strength of the first layer of between 12mT and 30mT.

The magnetic array is designed to work in synergy with the specific diamagnetic properties of the vitamin B3 active. The overall magnetic field strength of the magnetic array determines the amount of repulsive force induced in the vitamin B3 active and, as a consequence, the depth within skin to which the vitamin B3 active is driven, while the pitch of the magnetic poles determines the overall profile of the magnetic field. Use of such a magnetic array together with a composition containing a vitamin B3 active ensures that the maximum potential amount of vitamin B3 a) penetrates into a user's skin and b) is positioned at a layer of skin where it is likely to be most effective.

The magnetic array may further comprise a second layer of one or more dipolar pairs of alternating magnetic poles offset from the first layer at an angle of between 1° and 179°, the second layer of magnetic poles having a second layer pitch of between 1mm and 3.5mm, and a second layer magnetic field strength of between 8mT and 24mT, wherein the second layer magnetic field strength is less than or equal to the first layer magnetic field strength. Such a bi-directional magnetic array provides a more complex profile of magnetic field strength induced in the vitamin B3 active. Poles of the first and second layer constructively and destructively interfere with one another to reduce the areas of minimum magnetic flux density and ineffectual magnetic field strength.

The pitch of the second layer may be equal to or less than the pitch of the first layer. Additionally or alternatively, the overall magnetic field strength of the second layer is equal to or less than the overall magnetic field strength of the first layer. Typically the first layer is used to determine the maximum magnetic field strength, while the second layer smooths out the overall profile of the magnetic field.

The magnetic array has a proximal skin facing side and a distal side opposed thereto, wherein a magnetic return is provided at the distal side of the ferromagnetic substrate. The magnetic return is used to integrate the magnetic fields generated by each pole on that side of the substrate and reduces or eliminates the magnetic flux on that surface, thus directing the magnetic flux towards the skin facing side.

The method of constructing the magnetic array may involve separately magnetizing two layers of substrate with the two respective layers of poles and arranging the first and second layers of substrate in parallel such that the distal side of the second layer is adjacent the proximal side of the first layer.

Alternatively, the method of constructing the magnetic array may involve magnetizing a single ferromagnetic substrate with the poles of the first layer and subsequently magnetizing the same ferromagnetic substrate with poles of the second layer.

### BRIEF DESCRIPTION OF THE DRAWINGS

Embodiments of the invention will hereinafter be described, by way of example, with reference to the accompanying drawings, in which:
FIGS. 1A to 1D are perspective views of applicators of the skin care product described herein;
FIG. 2A shows schematically a conventional bar magnet having a north and a south pole;
FIG. 2B shows schematically a dipolar pair of magnets;
FIGS. 2C and 2D show schematically different arrangements of dipolar pairs in a magnetic array;
FIGS. 3A to 3E illustrate schematically the magnetization and corresponding magnetic field generated in a magnetic array of the skin care product described herein;
FIGS 4A and 4B illustrate schematically different ways of constructing a bi-directional magnetic array of the skin care product described herein;
FIG. 4C shows schematically a representation of the magnetic field generated by a bi-directional array;
FIG. 5 is a bar chart of the enhanced penetration of niacinamide using different magnetic arrays of the skin care product described herein;
FIG. 6 is a plot of the enhanced penetration of niacinamide using a magnetic array versus passive application with a finger.
FIG. 7 is a plot of the enhanced penetration of niacinamide using a magnetic array versus passive application with a non-magnetic applicator.

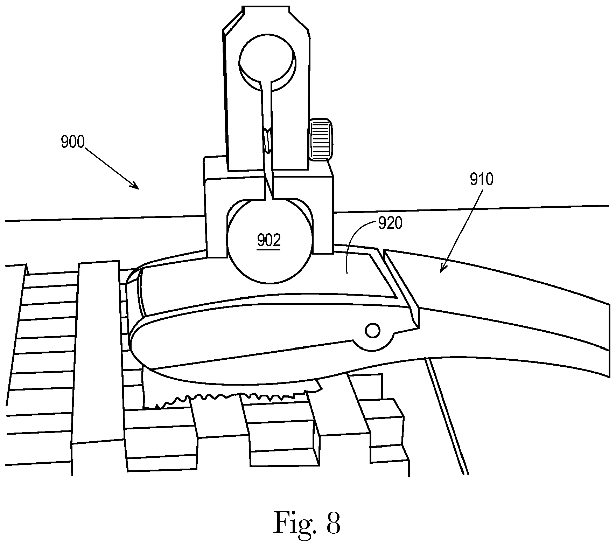

FIG. 8 illustrates the test setup for the Coefficient of Friction Method.

### DETAILED DESCRIPTION OF THE INVENTION

The skin care products disclosed herein exploit the unique diamagnetic property of certain skin care actives to enhance penetration of the actives into skin. Diamagnetism is the property of an object or material which causes it to create a magnetic field in opposition to an externally applied magnetic field, thus causing a repulsive effect. Surprisingly, it has been discovered that by pairing a specifically tailored magnetic array with a particular skin care active, penetration of the active into skin can be enhanced in a controllable way. Utilizing this discovery, it is possible to provide a cosmetic skin care product in which one or more skin care actives are delivered into skin to the point where they can provide a better skin care benefit than conventional skin care products.

The skin products disclosed herein provide enhanced penetration of skin care actives into skin. Methods of using the present skin products involve the use of a topical skin care composition in conjunction with an applicator that includes a magnetic array purposefully designed to enhance penetration of at least one skin care active in the composition.

### Definitions

"Apply" or "application", as used in reference to a composition, means to apply or spread the composition onto a surface of keratinous tissue.
"Derivative" refers to a molecule similar to that of another one, but differing from it in respect of a certain functional moiety.
"Disposed" refers to an element being located in a particular place or position relative to another element.
"Joined" means configurations whereby an element is directly secured to another element by affixing the element directly to the other element, and configurations whereby an element is indirectly secured to another element by affixing the element to intermediate member(s) that in turn are affixed to the other element.
"Keratinous tissue" refers to keratin-containing layers disposed as the outermost protective covering of mammals which includes, but is not limited to, skin, hair, nails, cuticles, etc.
"Magnetic field" and "magnetic flux density" are used interchangeably herein and refer to the vector field measured in teslas.
"Magnetic material" means a material that can be made into a permanent magnet.
"Pole" refers to the portion of a magnet that exhibits a higher magnetic flux density than the adjacent regions of the magnet. For example, a conventional bar magnet has two poles disposed at opposite ends where the magnetic flux density is highest.
"Regulating skin condition" means improving skin appearance and/or feel, for example, by providing a benefit, such as a smoother appearance and/or feel. Herein, "improving skin condition" means effecting a visually and/or tactilely perceptible positive change in skin appearance and feel. The benefit may be a chronic or acute benefit and may include one or more of the following: reducing the appearance of wrinkles and coarse deep lines, fine lines, crevices, bumps, and large pores; thickening of keratinous tissue (e.g., building the epidermis and/or dermis and/or sub-dermal layers of the skin, and where applicable the keratinous layers of the nail and hair shaft, to reduce skin, hair, or nail atrophy); increasing the convolution of the dermal-epidermal border (also known as the rete ridges), preventing loss of skin or hair elasticity, for example, due to loss, damage and/or inactivation of function skin elastin, resulting in such conditions as elastosis, sagging, loss of skin or hair recoil from deformation; reduction in cellulite; change in coloration to the skin, hair, or nails, for example, under-eye circles, blotchiness (e.g., uneven red coloration due to, for example, rosacea), sallowness, discoloration caused by hyperpigmentation, etc.
"Safe and effective amount" means an amount of a compound or composition sufficient to significantly induce a positive benefit, preferably a positive skin or feel benefit, including independently or in combinations the benefits disclosed herein, but low enough to avoid serious side effects (i.e., to provide a reasonable benefit to risk ratio), within the scope of sound judgment of the skilled artisan).
"Signs of skin aging" include, but are not limited to, all outward visibly and tactilely perceptible manifestations, as well as any macro- or micro-effects, due to keratinous tissue aging. These signs may result from processes which include, but are not limited to, the development of textural discontinuities such as wrinkles and coarse deep wrinkles, fine lines, skin lines, crevices, bumps, large pores, unevenness or roughness; loss of skin elasticity; discoloration (including under-eye circles); blotchiness; sallowness; hyperpigmented skin regions such as age spots and freckles; keratoses; abnormal differentiation; hyperkeratinization; elastosis; collagen breakdown, and other histological changes in the stratum corneum, dermis, epidermis, vascular system (e.g. telangiectasia or spider vessels), and underlying tissues (e.g., fat and/or muscle), especially those proximate to the skin.
"Skin" means the outermost protective covering of mammals that is composed of cells such as keratinocytes, fibroblasts and melanocytes. Skin includes an outer epidermal layer and an underlying dermal layer. Skin may also include hair and nails as well as other types of cells commonly associated with skin, such as, for example, myocytes, Merkel cells, Langerhans cells, macrophages, stem cells, sebocytes, nerve cells and adipocytes.
"Skin care" means regulating and/or improving a skin condition. Some nonlimiting examples include improving skin appearance and/or feel by providing a smoother, more even appearance and/or feel; increasing the thickness of one or more layers of the skin; improving the elasticity or resiliency of the skin; improving the firmness of the skin; and reducing the oily, skiny, and/or dull appearance of skin, improving the hydration status or moisturisation of the skin, improving the appearance of fine lines and/or wrinkles, improving skin exfoliation or desquamation, plumping the skin, improving skin barrier properties, improve skin tone, reducing the appearance of redness or skin blotches, and/or improving the brightness, radiancy, or translucency of skin.
"Skin care active" means a compound of combination of compounds that, when applied to skin, provide an acute and/or chronic benefit to skin or a type of cell commonly found therein. Skin care actives may regulate and/or improve skin or its associated cells (e.g., improve skin elasticity; improve skin hydration; improve skin condition; and improve cell metabolism).
"Skin care composition" means a composition that includes a skin care active and regulates and/or improves skin condition.

### Skin care product

The skin care product described herein includes a skin care composition containing one or more skin care actives, one of which is a vitamin B3 compound such as niacinamide, and an applicator that includes a magnetic array tailored to enhance delivery of the vitamin B3 compound into skin. The skin care composition and applicator may be packaged and sold together as a single product offering and/or they may be packaged separately to be sold individually. In some instances, the skin care composition and the applicator may be packaged in separate packages (e.g., in individual primary packages), which are then joined to one another or placed in a single secondary package. It may be desirable to include indicia on the applicator, the skin care composition and/or their respective package(s), which indicate that the magnetic properties of the array are tailored for use with the skin care composition, for example, to enhance penetration of one or more skin care actives. Indicia suitable for such use are not particularly limited and may include, for example, words, letters, numbers, shapes, colors, pictures and diagrams, which communicate to a consumer that the magnetic array is intended for use with the corresponding cosmetic composition. In some instances, the indicia may provide a non-verbal communication to a user that the magnetic array enhances penetration of a vitamin B3 compound.

### Applicator

The cosmetic skin care product described herein includes a suitable applicator for either applying a skin care composition to a target portion of skin or placing above and/or contacting a target portion of skin to which a skin care composition has already been applied. The form of the applicator may vary according to the intended target area of application on skin. For example, if the skin care composition is a whole body cream, then the applicator may be sized and/or shaped to apply the composition to larger surfaces and/or body parts, for example, the legs, arms, abdomen and/or back. In some instances, the skin care composition may be intended for use in smaller areas such as the face (e.g., cheeks, forehead, chin, nose, and peri-orbital regions). In such cases, the applicator may be correspondingly shaped and sized to use with smaller surface areas.

A magnetic array for incorporation into the present applicators may be configured to provide a skin contacting surface of the applicator (i.e., the magnetic array is disposed on the applicator such that it is brought into contact with a target skin surface when the applicator is used as intended). Thus, it is important for the magnetic material to be safe for topical use on skin, especially when used with a topical skin care composition. It may be desirable to select a magnetic material that provides a pleasant feel contacted with skin. For example, the magnetic array may be embedded in the applicator such that the applicator and the magnetic array are a unitary device that provides a smooth, comfortable surface when contacted with skin.

In some instances, the applicator may include an optional cover placed over at least a portion of the magnetic array and/or skin contacting surface, such that the cover becomes the skin contacting surface of the applicator. The cover may be permanently joined to the applicator, or the cover may be removable, detachable and/or replaceable. It may be desirable for the cover to have a coefficient of friction that is less than that of the magnetic substrate of the magnetic array, which can provide a more desirable user experience when applying a skin care composition with the applicator. In some instances, the cover may have a dry coefficient of friction (i.e., a coefficient of friction measured without using a composition) that is between 10 and 50% less than the magnetic substrate (e.g., 15%, 20%, 25%, 30%, 35%, 40%, or even 45% less) according to the Friction Test described in the example below. When used to apply a skin care composition, the cover may exhibit a coefficient of friction that is up to 10 times less than the magnetic array (e.g., between 2x and 10x less, 3x and 7x or even between 4x and 6x less).

The optional cover, when included, may be formed from a material that provides a skin contacting surface with better cooling properties than the magnetic substrate. For example, the cover may be formed of a material that has a high thermal conductivity, for example, at least 50 W/mK, 100 W/mK or 200 W/mK. Providing a cover with high thermal conductivity feels cool when contacted with skin. Because the thickness of the cover affects the distance that the magnetic flux density of the magnetic array extends, especially when formed from a non-magnetic material, it is important to ensure that the thickness of the cover does not undesirably inhibit the strength of the applied magnetic field. Suitable cover thicknesses are between 0.1 mm and 5 mm (e.g., between 0.2 and 4 mm, 0.5 and 3 mm, or even between 1 and 2 mm), for non-magnetic materials.

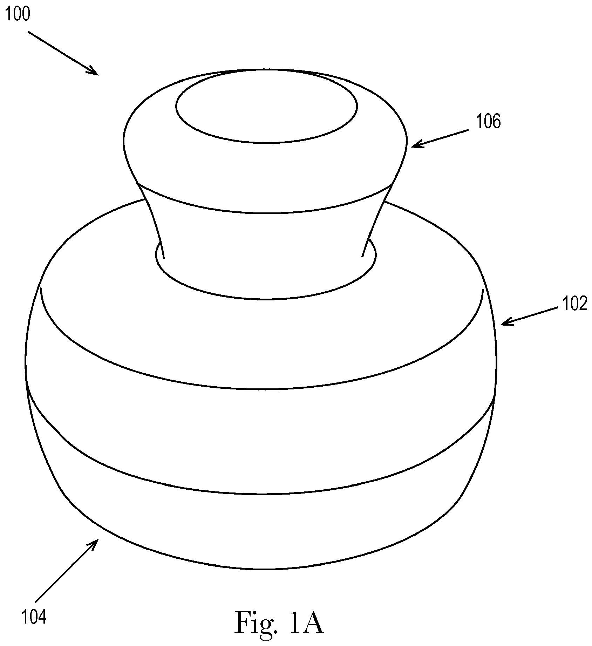

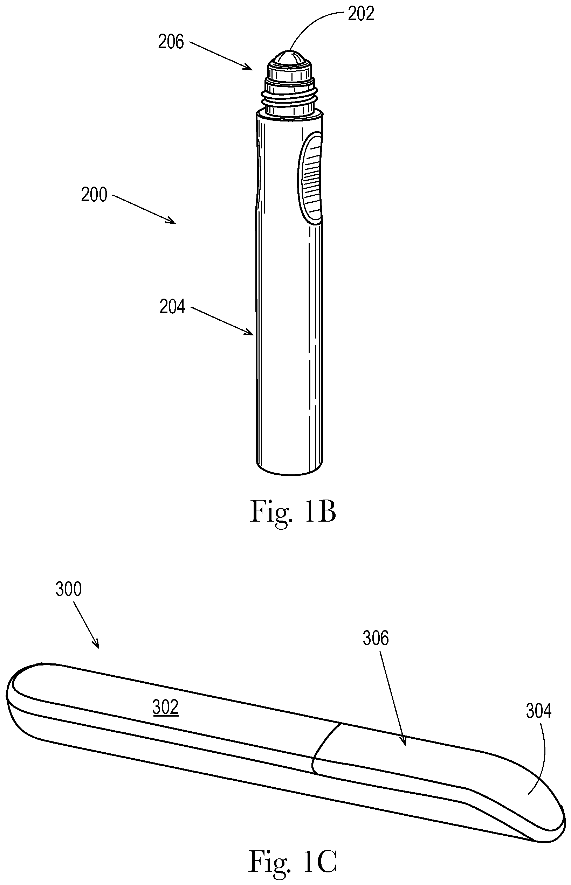

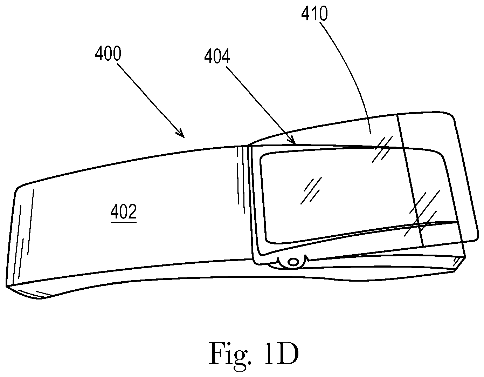

FIGS. 1A, 1B and 1C and 1D show non-limiting examples of applicators 100, 200, 300 and 400, respectively, for use in the present skin care products. The applicator 100 shown in FIG. 1A has a substantially cylindrical base 102 with a skin contact surface 104 extending across the base. A handle 106 extends from the base in a direction substantially perpendicular to the skin contact surface. A magnetic array is disposed inside the base (not shown), adjacent to and in parallel with the skin contact surface so that, in use, the magnetic array will be substantially parallel to any surface on which the applicator is used.

The applicator 200 shown in FIG. 1B has a rounded tip 202 that may be suitable for use around the eye. The rounded tip 202 may be integrally formed with a handle 204, or it may be formed as a ball held within a socket 206 at the end of the handle 204. A magnetic array (not shown), formed of a flexible substrate is disposed inside the rounded tip 202, such that as the tip 202 is rolled over a surface of skin, the magnetic array will be substantially parallel to the surface of skin. Thus, the tip 202 functions as a cover for magnetic array disposed within the tip 202.

The applicator 300 shown in FIG. 1C has an elongate handle 302 with a skin contacting tip 304 disposed on a skin facing side 306 of the applicator 300. A magnetic array (not shown) can be disposed inside the applicator 300, adjacent to and in parallel with the skin contacting tip 306, such that the magnetic array will be substantially parallel to any surface on which the applicator 300 is used.

The applicator 400 shown in FIG. 1D includes a removable cover 410. The cover 410 is joined to the skin facing side 404 of the applicator 400 and forms a skin contacting surface of the applicator 400, when used as intended. The cover 410 may be removed and/or replaced, as desired. In some instances, the cover 410 may be removed and reattached, for example, to facilitate cleaning the cover 410 and/or applicator 400. In some instances, the cover 410 may be disposable. For example, the cover 410 may be removed and discarded after 1 or more uses, but typically less than 10 uses, and replaced with a different cover. The cover 410 may be joined to the applicator 400 by any suitable means known in the art.

The applicators herein may be used to directly apply a skin care composition, or used to enhance penetration of skin care actives within a skin care composition after application of the skin care composition by some other means, for example, by finger application. For example, the applicator may be may be designed for movement across the skin's surface - either through manual operation or mechanical means (e.g., a vibrating device) or held in position stationary above a target area of skin to which a skin care composition has been applied. A vibrating device may include any mechanism, electrical or mechanical, adapted for reciprocal and/or rotational movement of the magnetic material. For example, the magnetic material may be associated with a drive mechanism that is capable of reciprocal movement.

Alternatively, the applicator may be made in the form of, for example, a leave on patch, in which case the applicator may be formed of a woven, flexible fabric. The patch may be formed with an adhesive section such that it can be adhered to a skin's surface following application of the skin care composition or the skin care composition may be contained within the patch.

### Magnetic array

The present applicator includes a magnetic array specifically tailored to provide improved penetration of a specific skin care active, such as a vitamin B3 compound. The magnetic array described herein uses selectively magnetized permanent magnets (i.e., materials that create their own persistent magnetic field without an extrinsic power source such as a battery) to generate a magnetic field. The magnets may be formed of any one of numerous known ferromagnetic substrates, including, but not limited to: an iron compound (e.g., a ferrite such as barium ferrite, magnetite, or mild steel), a cobalt material, a strontium material, a barium material, a nickel material, alloys and oxides of these, combinations thereof and the like. The material may have a metalloid component such as boron, carbon, silicon, phosphorous or aluminum. Rare earth material such as neodymium or samarium may also be used.

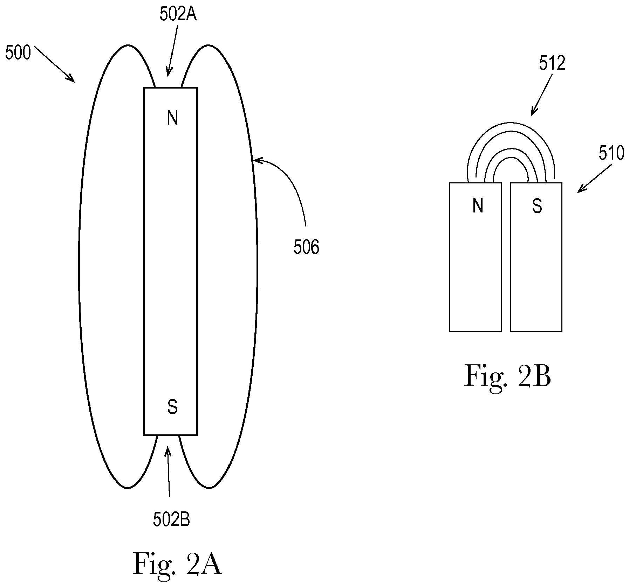

In a conventional bar magnet 500 such as the one illustrated in FIG. 2A, the magnetic field 506 extends between opposite ends 502A and 502B of the magnet 500. In contrast with a conventional bar magnet, the magnetic array(s) described herein are formed of one or more dipole pairs of magnetic elements where magnetic poles of opposite polarity (N and S) are positioned adjacent one another and the magnetic field extends between adjacent opposing poles. For purposes of visualization, a dipole pair 510 may be thought of as a conventional rod magnet that is cleaved at its center and the resulting sections brought together in a north-south (NS), side-by-side configuration.

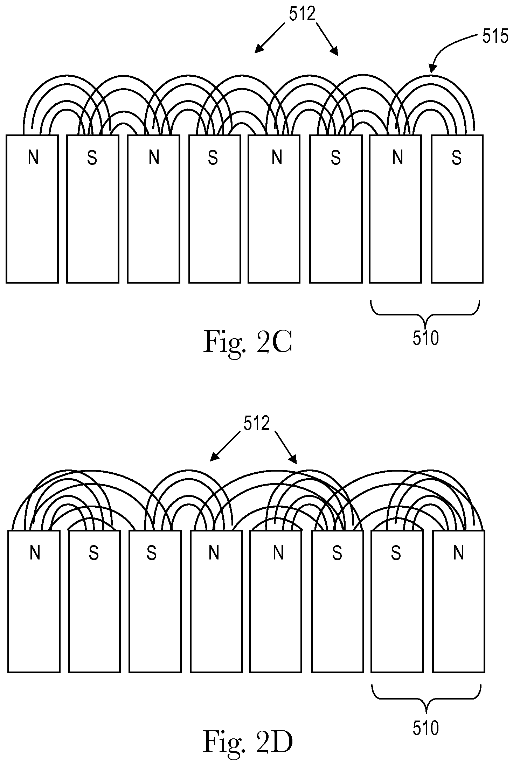

FIGS. 2B, 2C and 2D illustrate examples of magnetic arrays 510. Each of the magnetic arrays in FIGS. 2B, 2C and 2D include one or more dipole pairs 510. Magnetic fields 512 corresponding to the magnetic interaction of the dipole pairs 510 are represented by curved lines. FIG. 2B illustrates a magnetic array with one dipole pair 510 with a single corresponding magnetic field 512, whereas FIGS. 2C and 2D show multiple dipole pairs 510 arranged in series with multiple corresponding magnetic fields 512. When a magnetic array includes multiple dipole pairs 510, such as illustrated in FIGS. 2C and 2D, each dipole pair 510 can be in the same or a different orientation as that of the neighboring pair 510 (e.g., [NS][NS][NS] or [NS][SN][NS] as illustrated schematically in FIGS. 2C and 2D, respectively). In use, the magnetic fields 512 generated by the dipole pairs 510 will induce a magnetic field in a diamagnetic material. The induced magnetic field of the diamagnetic material interacts repulsively with the applied magnetic field 512 of the dipole pairs 510 regardless of the direction of the applied field 512 (i.e., north or south). The magnitude of the repulsive force between magnetic fields 512 of the dipole pairs 510 and the diamagnetic material is determined by the magnetic flux density of the corresponding dipole pair 510 and the diamagnetic susceptibility of the diamagnetic material, in this case the skin care active. Magnetic susceptibility is a dimensionless proportionality constant that indicates the degree of magnetization of a material in response to an applied magnetic field. A negative magnetic susceptibility generally indicates diamagnetism and may be referred to herein as diamagnetic susceptibility. Magnetic flux density is generally greatest at the mid-point 515 between the corresponding poles, and thus the strength of the magnetic field 512 will typically vary across the magnetic array depending on how the array is configured.

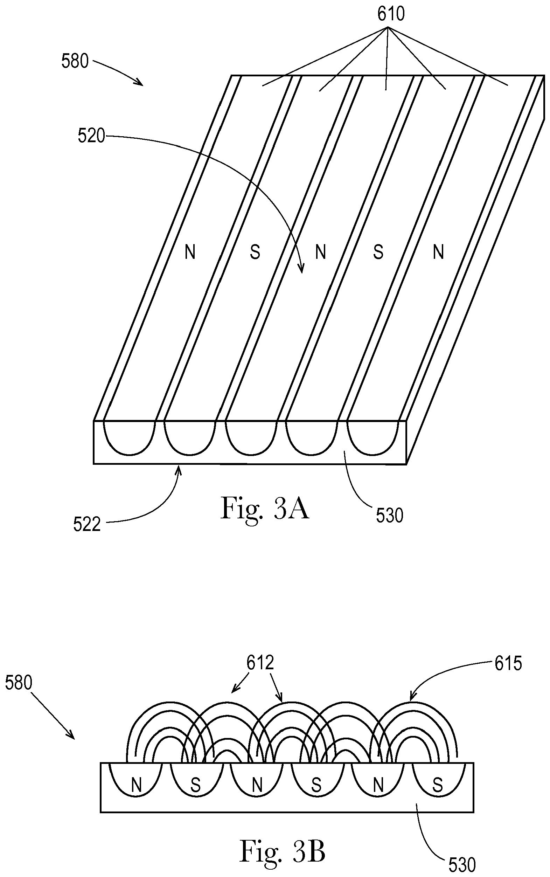

In practice, the substrate 580 used to form a magnetic array for use herein is typically not magnetized evenly throughout. As shown in FIG. 3A, each pole 610 extends from an upper skin facing side 520 of the substrate 580 towards an opposing underside 522 (i.e., through the thickness of the substrate 580). A magnetic return 530 is provided between each adjacent pole 610 and at the second side 522 of the substrate 580. The magnetic return 530 is an unmagnetized area used to integrate the magnetic fields 612 generated by each pole 610 on that side of the substrate 580 and reduce or eliminate the magnetic flux on the second side 522 of the substrate 580, instead diverting it towards the skin facing side 520. The resultant magnetic field 612 extends outward from the first side 520 of the substrate 580, in a direction substantially perpendicular to the surface of the substrate 580, and is strongest at the mid-point 615 between adjacent opposing poles 610.

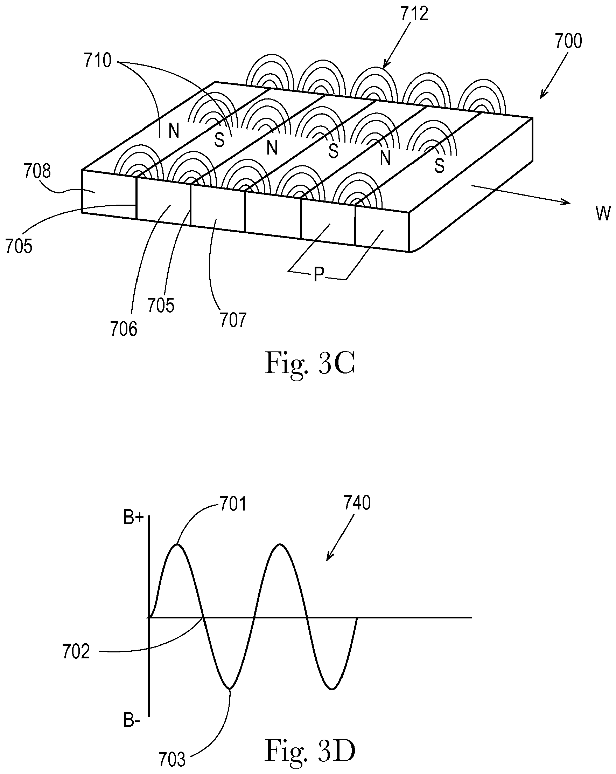

The magnetic array herein may be formed as a uni-directional array or a multi-directional array. FIG. 3C illustrates an example of a uni-directional array 700. The uni-directional array 700 has north (N) and south (S) poles 710 aligned in parallel to one another in a single layer, as shown in FIG. 3C. Adjacent poles 710 are separated from one another by a pole center-to-center distance P, which defines the pitch of the magnetic array 700.

FIG. 3D illustrates a portion of the magnetic field 712 generated by the magnetic array 700 of FIG. 3C in a direction W that is perpendicular to the alignment of the poles 710. The waveform 740 illustrated in FIG. 3D shows the magnitude of the magnetic field 712 varying regularly between +B and -B in a sinusoidal pattern, which corresponds to the difference in polarity (i.e., direction) of the magnetic field 712. The peaks 701 and troughs 703 of the waveform 740 correspond to the mid-point 705 between adjacent poles 710, and the inflection points 702 of the waveform 740 correspond to the centers of the poles 710. In other words, a first maximum magnetic flux density is represented by peak 701 occurs at a mid-point 705 between a first north pole 708 and an adjacent south pole 706, a minimum magnetic flux density represented by inflection point 702 occurs in the center of the south pole 706, and a second maximum magnetic flux density represented by trough 703 occurs at the mid-point 705 between the south pole 706 and a second north pole 707 adjacent the south pole 706.

The amplitude of the waveform 740 is determined by the choice of magnetic substrate, the thickness or depth of substrate that is magnetized and the distance from the center of a pole 710 to the edge of the pole 710. As the depth of magnetized area of a given substrate material increases, the maximum amplitude of the waveform 740 increases.

The frequency of the waveform 740 is determined by the pitch P of the array. A higher pitch P means that there are fewer magnetic flux density "maximums" per area of substrate, and thus a lower overall magnetic field strength for the array 700. However, a lower pitch P may result in respective poles 710 being packed too closely to one another for any single pole 710 to reach its maximum potential magnetic flux density.

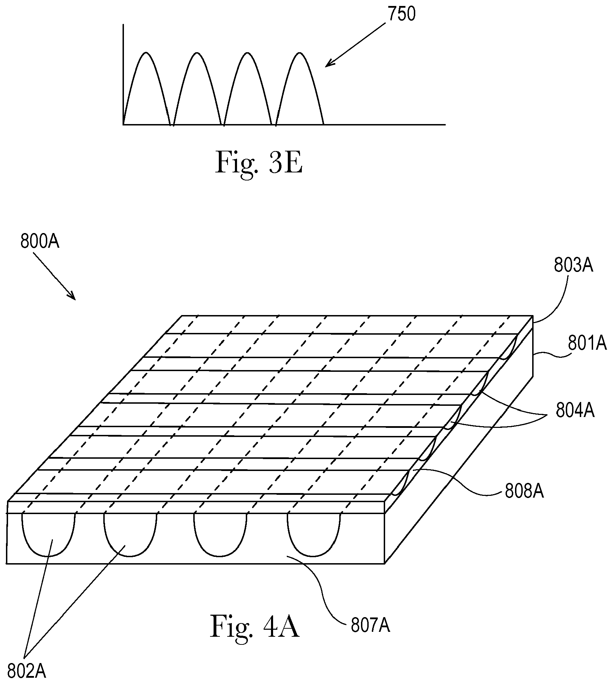

FIG. 3E is an illustration of a waveform 750 representing the repulsive force that would be experienced by a diamagnetic material exposed to the magnetic field 712 in FIG. 3D. As shown by the waveform 750, the induced magnetic field of a diamagnetic material is independent of the direction of the applied magnetic field 712, and thus the change in the magnitude of the repulsive force corresponds to the change in magnitude of the applied magnetic field 712.

In some instances, the magnetic array herein may be formed as a multi-directional array, e.g., a bi-directional array, in which multiple layers of parallel poles, which may be configured to play different roles, are juxtaposed at an angle relative to one another to provide multiple magnetic fields that constructively or destructively interfere with one another. For example, a first layer of poles may determine the maximum magnetic field strength, while a second set of poles smooths out the overall profile of the magnetic field, thereby reducing instances of minimum magnetic flux density and ineffectual magnetic field strength. Generally, in a multi-directional array, the magnetic flux density at any one point in the magnetic array will be determined by the combined magnetic flux density of poles of the different layers at that point. In some cases, this will lead to constructive interference where the resultant magnetic flux density at a point is greater than the magnetic flux density at that point for each individual layer. In other cases, the combination may lead to destructive interference where the resultant magnetic flux density at a point is less (sometimes zero) than the magnetic flux density at that point for each individual layer.

FIG. 4A illustrates an example of a bi-directional array 800A, wherein the first and second layers of poles 802A and 804A, respectively, are formed in two separate magnetic substrates 801A and 803A, which are subsequently juxtaposed at an angle offset from one another. The magnetic returns 807A and 808A of both substrates 601 and 603 are positioned to face in the same direction such that the magnetic field generated by both layers of poles 802A and 804A extends away from the magnetic array 800A in the same direction. The layers of poles 802A and 804A may be identical to one another (for example, having the same pitch between adjacent poles and the same maximum field strength), or the two layers 8802A and 8804A may vary in their specific parameters. Where the parameters of the two layers 802A and 804A vary, it is preferable for the layer that is proximal the target diamagnetic material (in FIG. 4A, the second layer 804A) to be formed of a thinner substrate than that of the distal layer (in FIG. 4A, the first layer 802A), otherwise the induced magnetic field of the diamagnetic material will be primarily based on the magnetic field strength of the proximal layer 804A.

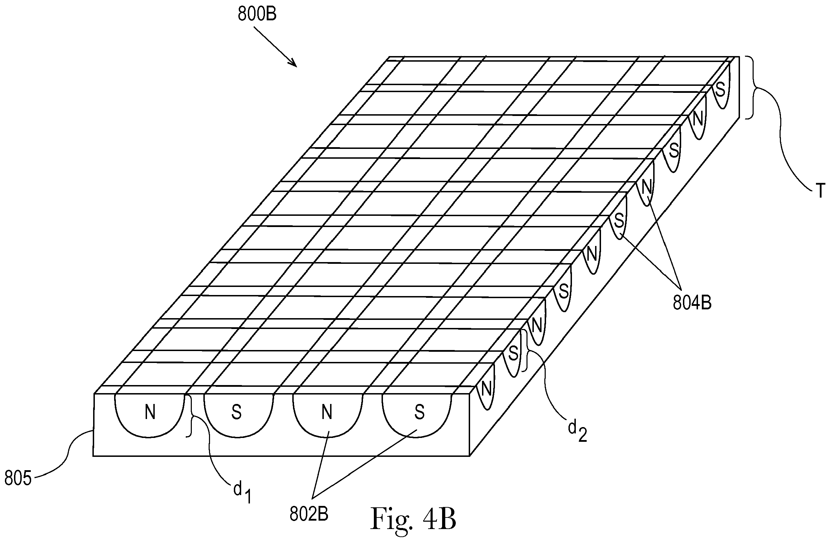

FIG. 4B illustrates an example in which the first layer of poles 802B and the second layer of poles 804B are formed in the same magnetic substrate 805. The configuration shown in FIG. 4B may be provided by first magnetizing the substrate 805 in one direction to form a first layer of parallel aligned north and south poles 802B, and then remagnetizing the substrate 805 in a different direction to form a second layer of parallel aligned north and south poles 804B to effectively form a woven pattern of poles. In this embodiment, the depth *d2* of poles in the second layer 804B is equal to or less than the depth d1 of poles in the first layer 802B. The depth *d1* of the first layer of poles 802B is typically determined by the thickness T of the magnetic substrate 805.

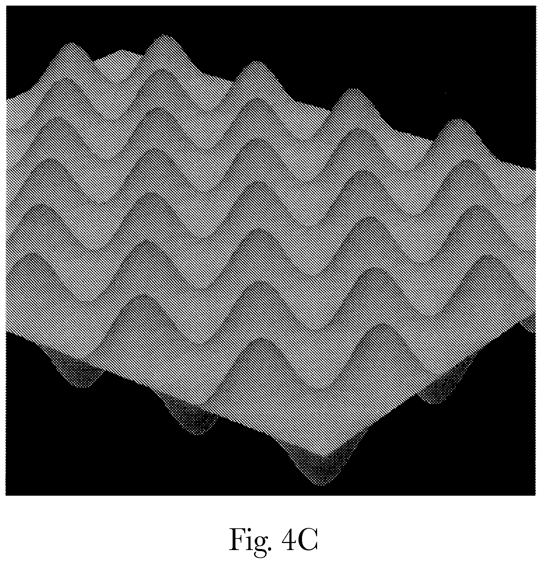

FIG. 4C illustrates a waveform representing the three-dimensional magnetic field of a bidirectional magnetic array. The induced magnetic field of a diamagnetic material is independent of the direction of the magnetic field, and thus all areas of positive and negative magnetic field strength will appear as a repulsive force to a diamagnetic material.

The combined overall magnetic field strength of a magnetic array can be measured after completion of the magnetization process using any known Gaussmeter. For bi-directional magnetic arrays made of two separate substrates, the overall magnetic field strength can be measured first for the respective layers and subsequently for the combined bi-directional magnetic array. In a bi-directional magnetic array, the overall magnetic field strength will approximately equate to the sum of the field strength of the individual layers.

Dipolar pairs of the magnetic substrate may be separated from adjacent dipolar pairs by a magnetically insulating material (i.e., a material with a relatively low magnetic permeability). In some instances, the magnetic elements may be arranged as individual segments or sections of magnetized ferromagnetic materials. Additionally or alternatively, the magnetic elements may be disposed in or on a solid or semi-solid substrate in which the required magnetic pattern is impressed upon the ferromagnetic particles or elements. The magnetic elements may be rigid elements within the applicator itself or disposed on a suitable substrate and joined to the applicator, for example, with an adhesive. In some instances, it may be desirable to embed the magnetic elements in a flexible matrix such as rubber or silicone and join the resultant array to a skin facing surface of the applicator.

When pairing a magnetic array with a skin care active such as a vitamin B3 compound, it is important for the magnetic field of the array to be tuned to interact with the diamagnetic susceptibility of the subject skin care active(s). If the magnetic field is improperly configured, for example, if the magnetic flux density is too low or the pitch between adjacent poles too great, there may be little to no magnetic field induced in the diamagnetic materials. Alternatively, if the magnetic flux density is too high, it may induce thermal noise and other forms of molecular entropy or disorder that act against the magnetic enhanced penetration of the skin care active. In some instances, even small departures from the proper configuration of the magnetic array may result in unsatisfactory penetration of the skin care actives.

In a particularly suitable example of a skin care product, a magnetic array is paired with a skin care composition that includes niacinamide. Niacinamide has a diamagnetic susceptibility of approximately -66. Magnetic arrays suitable for enhancing the penetration of niacinamide include uni-directional and/or bi-directional arrays that exhibit enhanced penetration of skin care actives with a diamagnetic susceptibility of between -50 and -80. The substrate may be formed of strontium ferrite powder impregnated in a polyvinyl chloride PVC base. A suitable uni-directional array may have a thickness of between 0.2mm, 0.3mm, 0.4mm or 0.5mm and 0.6mm, 0.7mm, 0.8mm, 0.9mm or 1 mm, a pitch (center to center distance between poles) of 1mm, 1.5mm or 2mm to 2.5mm, 3mm or 3.5mm between adjacent poles, leading to an overall magnetic field strength of between 12mT, 14mT, 15mT, 17.5mT or 20mT to 22.5mT, 25mT, 28mT or 30mT. In a particularly suitable example of a uni-directional magnetic array, the magnetic array has an overall magnetic field strength of approximately 23mT, a thickness of 0.6mm and a pitch of about 2.1mm (e.g., 12 poles per 25.4mm).

An example of a suitable bi-directional array for enhancing penetration of a vitamin B3 compound into skin may have a first layer thickness of between 0.2mm, 0.3mm, 0.4mm or 0.5mm and 0.6mm, 0.7mm, 0.8mm, 0.9mm or 1 mm, a first layer pitch (center to center distance between poles) of 1mm, 1.5mm or 2mm to 2.5mm, 3mm or 3.5mm between adjacent poles, leading to a first layer magnetic field strength of between 12mT, 14mT, 17.5mT or 20mT to 22.5mT, 25mT, 28mT or 30mT, and a second layer thickness of between 0.05mm, 0.1mm, 0.15mm or 0.2mm and 0.25mm, 0.3mm, 0.4mm or 0.6mm, a second layer pitch of 1mm, 1.25mm or 1.5mm to 2.5mm, 3mm or 3.5mm between adjacent poles, leading to a second layer magnetic field strength of between 8mT, 10mT, 12mT, 14mT or 16mT and 18mT, 20mT, 22mT or 24mT. The overall magnetic field strength of the bi-directional array may be between 14mT and 30mT. Typically, in a bi-directional array, the second layer magnetic field strength will be less than or equal to the first layer magnetic field strength and/or second layer pitch will be equal to or less than the first layer pitch. The first and second layers of the bi-directional array in this example may be angularly offset by between 1, 30, 45, 60 or 90 degrees and 120, 140, 160 or 179 degrees.

In a particularly suitable example of a bi-directional array, the magnetic array has an overall magnetic field strength of 27mT, a first layer thickness of 0.6mm, a first layer pitch of 2.1mm (12 poles per 25.4mm) and a second layer thickness of 0.2mm and second layer pitch of 1.49mm (17 poles per 25.4mm).

The first layer of a bi-directional array may be formed of a uni-directional array.

### Skin Care Composition

A skin care composition of the present invention may be applied to mammalian keratinous tissue, in particular to human skin. The cosmetic compositions may take various forms such as, for example, solutions, suspensions, lotions, creams, gels, toners, sticks, pencils, sprays, aerosols, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks, cosmetics (e.g. foundations, eye liners, eye shadows), and the like.

Skin care compositions may include a first skin care active such as a vitamin B3 compound, for example niacin or niacinamide. As used herein, "vitamin B3 compound" means a compound having the formula: 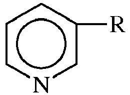 wherein R is - CONH₂ (i.e., niacinamide), - COOH (i.e., nicotinic acid) or - CH2OH (i.e., nicotinyl alcohol); derivatives thereof; and salts of any of the foregoing.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye, i.e., the ester is non-rubifacient). Non-vasodilating esters of nicotinic acid include tocopherol nicotinate and inositol hexanicotinate; tocopherol nicotinate is preferred.

Other derivatives of the vitamin B₃ compound are derivatives of niacinamide resulting from substitution of one or more of the amide group hydrogens. Nonlimiting examples of derivatives of niacinamide useful herein include nicotinyl amino acids, derived, for example, from the reaction of an activated nicotinic acid compound (e.g., nicotinic acid azide or nicotinyl chloride) with an amino acid, and nicotinyl alcohol esters of organic carboxylic acids (e.g., C1 - C18). Specific examples of such derivatives include nicotinuric acid (C8H8N2O3) and nicotinyl hydroxamic acid (C6H6N2O2), which have the following chemical structures:
nicotinuric acid: 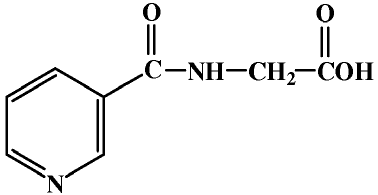
nicotinyl hydroxamic acid:

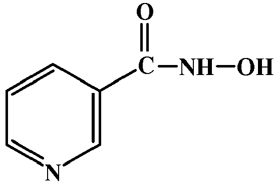

Exemplary nicotinyl alcohol esters include nicotinyl alcohol esters of the carboxylic acids salicylic acid, acetic acid, glycolic acid, palmitic acid and the like. Other non-limiting examples of vitamin B3 compounds useful herein are 2-chloronicotinamide, 6-aminonicotinamide, 6-methylnicotinamide, n-methyl-nicotinamide, n,n-diethylnicotinamide, n-(hydroxymethyl)-nicotinamide, quinolinic acid imide, nicotinanilide, n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Examples of the above vitamin B3 compounds are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

One or more vitamin B3 compounds may be used herein. Preferred vitamin B3 compounds are niacinamide and tocopherol nicotinate. Niacinamide is more preferred.

When used, salts, derivatives, and salt derivatives of niacinamide are preferably those having substantially the same efficacy as niacinamide.

Salts of the vitamin B3 compound are also useful herein. Nonlimiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- C1 - C18 carboxylic acid salts, e.g., acetate, salicylate, glycolate, lactate, malate, citrate, preferably monocarboxylic acid salts such as acetate). These and other salts of the vitamin B3 compound can be readily prepared by the skilled artisan, for example, as described by W. Wenner, "The Reaction of L-Ascorbic and D-Iosascorbic Acid with Nicotinic Acid and Its Amide", J. Organic Chemistry, Vol. 14, 22-26 (1949). Wenner describes the synthesis of the ascorbic acid salt of niacinamide.

In a preferred embodiment, the ring nitrogen of the vitamin B3 compound is substantially chemically free (e.g., unbound and/or unhindered), or after delivery to the skin becomes substantially chemically free ("chemically free" is hereinafter alternatively referred to as "uncomplexed"). More preferably, the vitamin B3 compound is essentially uncomplexed. Therefore, if the composition contains the vitamin B3 compound in a salt or otherwise complexed form, such complex is preferably substantially reversible, more preferably essentially reversible, upon delivery of the composition to the skin. For example, such complex should be substantially reversible at a pH of from about 5.0 to about 6.0. Such reversibility can be readily determined by one having ordinary skill in the art.

More preferably the vitamin B3 compound is substantially uncomplexed in the composition prior to delivery to the keratinous tissue. Exemplary approaches to minimizing or preventing the formation of undesirable complexes include omission of materials which form substantially irreversible or other complexes with the vitamin B3 compound, pH adjustment, ionic strength adjustment, the use of surfactants, and formulating wherein the vitamin B3 compound and materials which complex therewith are in different phases. Such approaches are well within the level of ordinary skill in the art.
Thus, in a preferred embodiment, the vitamin B3 compound contains a limited amount of the salt form and is more preferably substantially free of salts of a vitamin B3 compound. Preferably the vitamin B3 compound contains less than about 50% of such salt, and is more preferably essentially free of the salt form. The vitamin B3 compound in the compositions hereof having a pH of from about 4 to about 7 typically contain less than about 50% of the salt form.
The vitamin B3 compound may be included as the substantially pure material, or as an extract obtained by suitable physical and/or chemical isolation from natural (e.g., plant) sources. The vitamin B3 compound is preferably substantially pure, more preferably essentially pure.

In some examples, the cosmetic compositions may have a concentration of a vitamin B3 compound, by weight of the cosmetic composition, of greater than 0.0005%, 0.00056%, 1%, 2%, 3%, 4%, or 5% and/or less than 11%, 10%, 8%, or 6%.

The topical application of niacinamide may be associated with a variety of cosmetic skin care benefits. These may include: i) normalization of age associated depletions of nicotinamide coenzymes in skin, ii) up-regulation of epidermal ceramide synthesis with concurrent epidermal barrier benefits, iii) protection against damage produced by UV irradiation, iv) inhibition of the transfer of melanosomes from melanocytes to keratinocytes (thereby providing a potential skin tone benefit), and reduction in sebaceous lipogenesis. Thus, in certain instances, it may be desirable to include niacinamide in the cosmetic composition in order to improve the appearance of aging/photo-damaged skin.

The cosmetic compositions may also comprise a dermatologically acceptable carrier (which may also be referred to as a "carrier") within which the vitamin B3 compound is incorporated to enable the compound and optional other ingredients to be delivered to the skin. The carrier may contain one or more dermatologically acceptable solid, semi-solid or liquid fillers, diluents, solvents, extenders components, materials and the like. The carrier may be solid, semi-solid or liquid. The carrier may be provided in a wide variety of forms. Some non-limiting examples include simple solutions, (aqueous or oil based), emulsions, and solid forms (e.g., gels, sticks, flowable solids, amorphous materials).

The carriers may contain one or more dermatologically acceptable, hydrophilic diluents. Hydrophilic diluents include water, organic hydrophilic diluents such as lower monovalent alcohols (e.g., C1 -C4) and low molecular weight glycols and polyols, including propylene glycol, polyethylene glycol (e.g., molecular weight 200-600 g/mole), polypropylene glycol (e.g., molecular weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof.

Carriers may also be in the form of an emulsion, such as oil-in-water emulsions, water-in-oil emulsions, and water-in-silicone emulsions. An emulsion may generally be classified as having a continuous aqueous phase (e.g., oil-in-water and water-in-oil-in-water) or a continuous oil phase (e.g., water-in-oil and oil-in-water-in-oil). The oil phase may comprise silicone oils, non-silicone oils such as hydrocarbon oils, esters, ethers, and the like, and mixtures thereof. The aqueous phase may comprise water, such as a solution as described above. However, in other embodiments, the aqueous phase may comprise components other than water, including but not limited to water-soluble moisturizing agents, conditioning agents, anti-microbials, humectants and/or other water-soluble skin care actives.

Various cosmetic treatments may be employed. Skin surfaces of the most concern tend to be those not typically covered by clothing such as facial skin surfaces, hand and arm skin surfaces, foot and leg skin surfaces, and neck and chest skin surfaces. In particular, facial skin surfaces, including the forehead, peri-oral, chin, peri-orbital, nose, and/or cheek skin surfaces, may be treated with the cosmetic compositions described herein.

### Methods of Use

The skin care product described herein may be used for applying the skin care composition to one or more skin surfaces as part of a user's daily routine. A consumer may use the skin care product by dispensing a desired amount of the skin care composition onto the applicator and then, using the skin contact surface of applicator, applying the composition to a target area of the person's skin. In doing so, the magnetic array located within the applicator is able to act together with the diamagnetically susceptible material within the skin care composition to increase the volume of skin care active that penetrates into skin. The skin care composition may be applied to the applicator manually by the user (for example, by using the applicator to scoop some of the composition out of a tub) and/or the composition may be held in a reservoir provided in the applicator and dispensed automatically onto the skin contact surface of the applicator.

Additionally or alternatively, the skin care composition may be applied directly to a user's skin surface in a normal manner (i.e. by finger application) and the applicator subsequently swept over the target area of skin.

The skin care composition may be intended primarily for use on facial skin surfaces, including one or more of the cheek, forehead and peri-orbital areas of the face.

### EXAMPLES

The following examples are given solely for the purpose of illustration and are not to be construed as limiting the invention, as many variations thereof are possible.

### EXAMPLE 1 - VITAMIN B3 in vivo skin penetration study #1

An *in vivo* skin penetration study was conducted to establish the effect of using a skin care product of the present invention by applying a skin care composition comprising a vitamin B3 active (in this case, niacinamide) with an applicator comprising a magnetic array. The study used 10 active study sites (composition applied to target skin surface using an applicator containing a magnetic array of the present invention) versus 10 passive study sites (composition applied to target skin surface using traditional finger application). In this example, the level of vitamin B3 compound present in the extract from each tape strip was measured using HPLC and the results normalized to the protein level measured on the tape strip. While passive delivery is accomplished using a finger, it is to be appreciated that passive delivery may also be accomplished using an applicator or other device that does not include a magnetic array tailored to enhance penetration of niacinamide.

### Tape Stripping Method

This method provides a suitable means of measuring the amount of skin care active present in skin, and comparing active versus passive application of the skin care active. Two identical circular areas of 7.9cm² were marked on the volar forearms of volunteers. A measured dose (approximately 9mg) of the skin care formulation (ingredients shown in Table 1) was applied to the delineated areas using a screw actuated syringe. Active application was carried out using purpose made applicators having a metallic aluminium skin contact surface behind which was the magnetic array. The cream was spread evenly across the entire delineated region using the applicator in a sweeping motion with a fixed speed of approximately 3.5cm/s to mimic normal finger application. Passive application was performed using the tip of a finger in a sweeping motion identical to that of application using the applicator. The application period was 30 seconds during which time visual inspection was used to ensure even distribution and absorption of the formulation by skin. The application area was then left uncovered for a further 30 minutes to ensure complete absorption. The application area was then wiped thoroughly to remove any surface material, followed by carrying out tape stripping.

The Tape Stripping Method can be carried out using commercial pre-cut 22.1mm tape stripping adhesive discs (D-SQUAME brand tape strips from Cuderm Corporation or equivalent) with an adhesive area of 3.8cm². A 22.1mm diameter circular region was marked at the center of the application area. A tape stripping adhesive disc was placed over the marked area and even pressure applied using a neoprene roller, rolled ten times over the surface. The adhesive disc was removed from the skin surface in a single pulling motion using manual tweezers. To ensure even removal of the stratum corneum, subsequent discs were removed in a north, south, east and west orientation. Each adhesive disc was non-destructively analyzed for relative protein content using the SquameScan™ 850 instrument (Heiland Electronics Wetzlar Germany). The adhesive disc was then immediately placed into a glass vial containing extraction solvent in preparation for subsequent analysis. Solvent extractions are conducted on each tape strip using conventional extraction methods, which are well known to those of ordinary skill in the art, and measuring the amount of niacinamide present in the extract, for example, by high performance liquid chromatography ("HPLC") and/or mass spectrometry.

The procedure was repeated for the remaining nine strips. An additional strip was obtained from outside the area of application of the skin care formulation to serve as a blank sample.

Delivery of the niacinamide is said to be enhanced when a ratio of active to passive delivery, as determined according to the Tape Stripping Method, is greater than 1. In other words, if active application of the skin care composition yields more niacinamide than the corresponding passive application, then delivery is said to be enhanced. The active and corresponding passive application values may be compared individually (e.g., single tape strip comparison) or as group of two or more values (e.g., the sum total and/or average values of tape strips 8, 9, and 10 for the active and passive applications may be compared to determine if penetration was enhanced). The tailored magnetic arrays herein enhance delivery of vitamin B3 compounds such as niacinamide. Enhanced delivery may be from 1.5x to 20x (2x, 2.5, 3x, 3.5x, 4x, 4.5x, 5x, 5.5x, 6x, 6.5x, 7x, 7.5x, 8x, 8.5x, 9x, 9.5x or even 10x or more).

The above described process was repeated for 5 different magnetic arrays, as follows:
1. Uni-directional magnetic array:
   a. Substrate: strontium ferrite impregnated in polyvinyl chloride
   b. Thickness of substrate: 0.6mm
   c. Pitch between poles: 2.13mm (12 poles per 25.4mm/0.47 poles per mm)
   d. Overall magnetic field strength: 21.5mT
2. Bi-directional magnetic array (two layers formed on different substrates):
   1^{st}-layer:
      a. Substrate: strontium ferrite impregnated in polyvinyl chloride
      b. Thickness of substrate: 0.6mm
      c. Pitch between poles: 2.13mmmm (12 poles per 25.4mm/0.47 poles per mm)
   2^{nd}-layer:
      d. Substrate: strontium ferrite impregnated in polyvinyl chloride
      e. Thickness of substrate: 0.4mm
      f. Pitch between poles: 1.49mm (17 poles per 25.4mm/0.67 poles per mm)
   Magnetic array:
      g. Angle between 1^{st} and 2^{nd} layer: 90°
      h. Overall magnetic field strength: 27 mT
3. Bi-directional magnetic array (two layers formed on different substrates):
   1^{st}-layer:
      a. Substrate: strontium ferrite impregnated in polyvinyl chloride
      b. Thickness of substrate: 0.6mm
      c. Pitch between poles: 2.13mm (12 poles per 25.4mm/0.47 poles per mm)
   2^{nd}-layer:
      d. Substrate: strontium ferrite impregnated in polyvinyl chloride
      e. Thickness of substrate: 0.2mm
      f. Pitch between poles: 1.49mm (17 poles per 25.4mm/0.67 poles per mm) Magnetic array:
      g. Angle between 1^{st} and 2^{nd} layer: 90°
      h. Overall magnetic field strength: 24mT
4. Bi-directional magnetic array (two layers formed on different substrates):
   1^{st}-layer:
      a. Substrate: strontium ferrite impregnated in polyvinyl chloride
      b. Thickness of substrate: 0.4mm
      c. Pitch between poles: 3.18mm (8 poles per 25.4mm/0.31 poles per mm)
   2^{nd}-layer:
      d. Substrate: strontium ferrite impregnated in polyvinyl chloride
      e. Thickness of substrate: 0.4mm
      f. Pitch between poles: 3.18mm (8 poles per 25.4mm/0.31 poles per mm)
   Magnetic array:
      g. Angle between 1^{st} and 2^{nd} layer: 90°
      h. Overall magnetic field strength: 27mT
5. Bi-directional magnetic array (two layers formed on different substrates):
   1^{st}-layer:
      a. Substrate: strontium ferrite impregnated in polyvinyl chloride
      b. Thickness of substrate: 0.6mm
      c. Pitch between poles: 2.13mm (12 poles per 25.4mm/0.47 poles per mm)
   2^{nd}-layer:
      d. Substrate: strontium ferrite impregnated in polyvinyl chloride
      e. Thickness of substrate: 0.2mm
      f. Pitch between poles: 1.02mm (25 poles per 25.4mm/0.98 poles per mm) Magnetic array:
         g. Angle between 1^{st} and 2^{nd} layer: 90°
         h. Combined overall magnetic field strength: 22.5mT

**Table 1: Data of niacinamide penetration using the strip test method described above for Magnetic Arrays 1 to 5.**

| **Strip** | **Passive** | **Array 1 12p6** | **Passive** | **Array 2 12p6x17p4** | **Passive** | **Array 3 12p6x17p2** | **Passive** | **Array 4 8p4x8p4** | **Passive** | **Array 5 12p6x25p2** |
|---|---|---|---|---|---|---|---|---|---|---|
| **1** | 0.00845 | 0.013616 | 0.017843 | 0.021986 | 0.011035 | 0.024631 | 0.01069 | 0.020344 | 0.011784 | 0.009747 |
| **2** | 0.006178 | 0.014602 | 0.011586 | 0.017089 | 0.008634 | 0.018679 | 0.005361 | 0.012064 | 0.004857 | 0.007167 |
| **3** | 0.007764 | 0.015825 | 0.011584 | 0.015866 | 0.008218 | 0.024277 | 0.004879 | 0.009105 | 0.006244 | 0.006392 |
| **4** | 0.007248 | 0.014348 | 0.012865 | 0.0197 | 0.009459 | 0.025658 | 0.006818 | 0.009201 | 0.004351 | 0.008333 |
| **5** | 0.005499 | 0.008666 | 0.012146 | 0.01303 | 0.007459 | 0.019737 | 0.004964 | 0.007965 | 0.00438 | 0.003562 |
| **6** | 0.005078 | 0.010503 | 0.006244 | 0.012447 | 0.004416 | 0.016562 | 0.004431 | 0.005629 | 0.004389 | 0.004891 |
| **7** | 0.004794 | 0.007572 | 0.005434 | 0.010297 | 0.004242 | 0.012092 | 0.003876 | 0.004599 | 0.003079 | 0.002622 |
| **8** | 0.003777 | 0.005703 | 0.004632 | 0.005926 | 0.003534 | 0.008018 | 0.002613 | 0.004901 | 0.002371 | 0.001721 |
| **9** | 0.003581 | 0.005068 | 0.004075 | 0.006197 | 0.002609 | 0.007265 | 0.002511 | 0.003496 | 0.001491 | 0.003057 |
| **10** | 0.003353 | 0.003065 | 0.004453 | 0.008301 | 0.002032 | 0.005458 | 0.002165 | 0.003261 | 0.001297 | 0.001653 |
| **Sum 6-10** | 0.020584 | 0.031910 | 0.024839 | 0.043168 | 0.016833 | 0.049395 | 0.015595 | 0.021886 | 0.012627 | 0.013943 |
| **Enhancement** | | x1.55 | | X1.74 | | X2.93 | | X1.40 | | X1.10 |

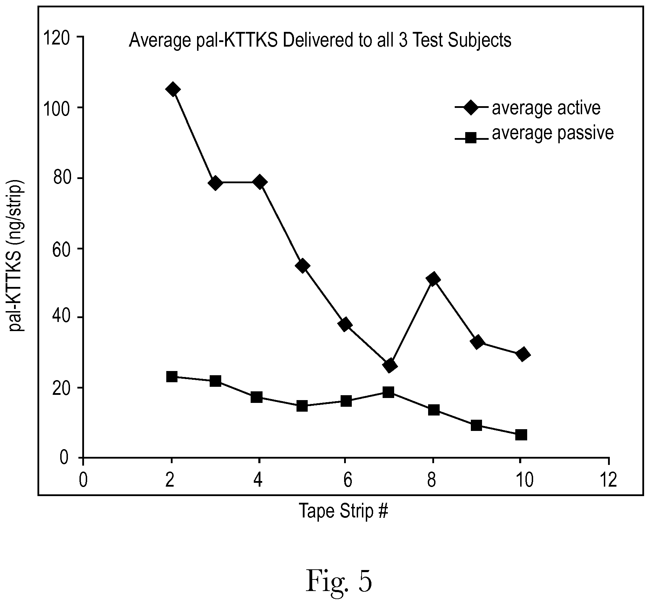

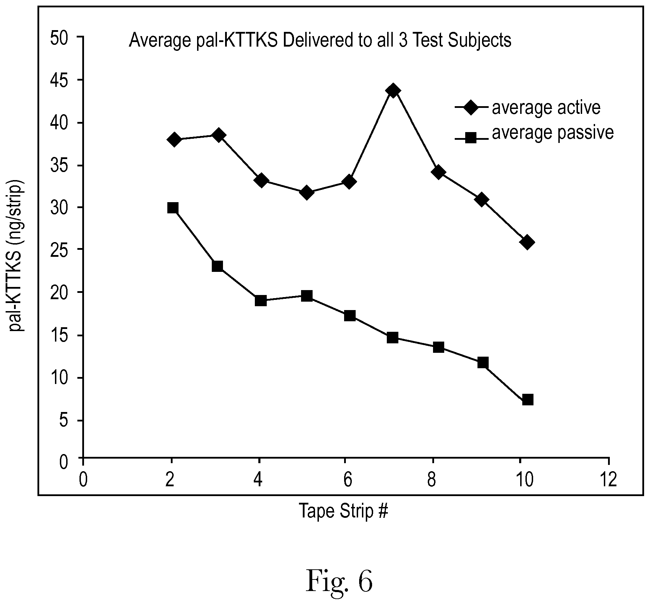

Magnetic array 1 above is a uni-directional array, magnetic arrays 2 to 5 are all bi-directional arrays. From this data and the corresponding bar chart shown in FIG. 5, it can be seen that in each case, use of an applicator with a magnetic array resulted in increased penetration of niacinamide vs passive, finger application. The best results for increased penetration of niacinamide were seen in Example 3 (bi-directional array 12p6x17p2), where approximately 3 times the amount of niacinamide was seen at all levels of skin (i.e. strips 1 through to 10), as shown in FIG. 6.

### Example 3 - Vitamin B₃ In Vivo Skin Penetration Study #2

This *in vivo* skin penetration study compares the penetration of niacinamide into skin when a niacinamide-containing composition is applied with a magnetic applicator (active application) versus application with a non-magnetic applicator (passive application). In this example, 5 test subjects (A to E in Tables 13 and 14) were selected. 18mg of a composition containing niacinamide (Olay® Deep Wrinkle Treatment® brand skin cream available from the Procter & Gamble Company, Cincinnati, Ohio) was applied to two 3 cm x 3 cm test sites on the inner forearms of each test subject using the applicator illustrated in FIG. 1C. The applicator used for active application included Array #8 from Table 2. The applicator used for passive application was the same as the one used for active application except without the magnetic array. Each forearm included an active application test site and a passive application site for a total of 10 active test sites and 10 passive sites. The application time was 30 seconds with a speed of motion of approximately 3 cm per second, equating to a gentle rubbing action. Application of the composition was followed by a 30 minute absorption period. The results of the passive and active application are shown in Tables 13 and 14 below. Penetration of niacinamide was determined according to the Tape Stripping Method. The level of niacinamide recovered from each tape strip was measured using HPLC.

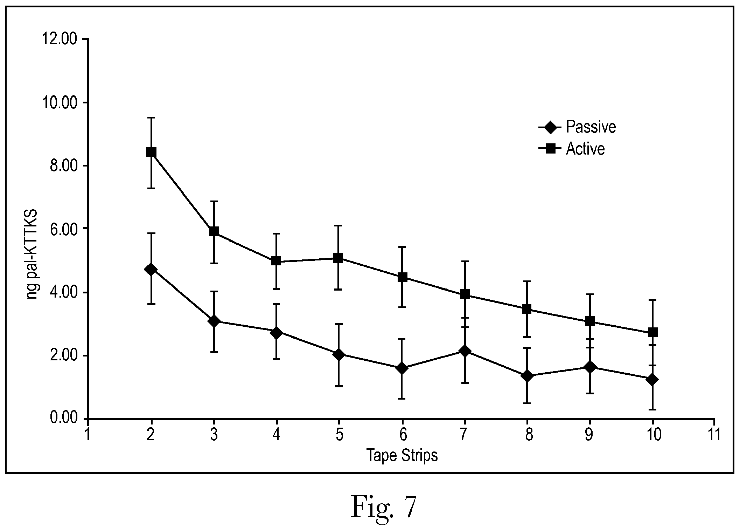

Tables 13 and 14 show the amount of niacinamide recovered from each tape strip. Table 13 shows the results of applying the composition using a non-magnetic applicator, and Table 14 shows the results of applying the composition with a magnetic applicator configured to enhance penetration of niacinamide. The average value across all test sites is shown in the second to last cell of the row. The standard error of the mean (SEM) is shown in the last column of Tables 13 and 14. The SEM is calculated by dividing the standard deviation by the square root of the number of test sites. The active versus passive application results from Tables 13 and 14 are graphically illustrated in FIG. 7.

**Table 13 - Passive Application**

| | Test Subject A | | Test Subject B | | Test Subject C | | Test Subject D | | Test Subject E | | | |
|---|---|---|---|---|---|---|---|---|---|---|---|---|
| | Site 1 | Site 2 | Site 1 | Site 2 | Site 1 | Site 2 | Site 1 | Site 2 | Site 1 | Site 2 | Avg | SEM |
| 1 | 2.62 | 3.07 | 2.86 | 3.16 | 5.69 | 4.14 | 6.28 | 0.54 | 7.00 | 3.31 | 3.87 | 0.62 |
| 2 | 2.66 | 2.71 | 2.52 | 4.24 | 7.80 | 5.47 | 7.28 | 1.84 | 2.88 | 3.81 | 4.12 | 0.66 |
| 3 | 3.51 | 6.33 | 3.77 | 6.64 | 7.64 | 6.14 | 9.62 | 2.43 | 5.36 | 5.43 | 5.69 | 0.67 |
| 4 | 4.22 | 8.53 | 4.24 | 7.48 | 7.15 | 5.87 | 11.29 | 1.29 | 8.91 | 2.79 | 6.18 | 0.97 |
| 5 | 4.57 | 6.08 | 2.74 | 6.35 | 6.21 | 5.61 | 7.10 | 2.27 | 4.36 | 3.79 | 4.91 | 0.51 |
| 6 | 4.37 | 6.69 | 1.97 | 5.29 | 7.95 | 4.56 | 6.46 | 2.62 | 3.85 | 3.42 | 4.72 | 0.60 |
| 7 | 3.57 | 4.35 | 1.87 | 3.82 | 6.89 | 5.41 | 0.19 | 2.67 | 4.47 | 3.37 | 3.66 | 0.59 |
| 8 | 2.65 | 2.85 | 1.56 | 4.65 | 5.34 | 2.94 | 5.75 | 1.88 | 3.71 | 2.92 | 3.43 | 0.45 |
| 9 | 2.95 | 2.24 | 2.00 | 3.78 | 6.70 | 2.79 | 7.20 | 2.15 | 3.70 | 2.37 | 3.59 | 0.59 |
| 10 | 1.58 | 1.54 | 1.31 | 4.53 | 5.34 | 2.42 | 6.19 | 0.71 | 6.10 | 2.24 | 3.20 | 0.67 |
| Sum 2 - 10 | 30.09 | 41.32 | 21.97 | 46.79 | 61.02 | 41.20 | 61.08 | 17.87 | 43.34 | 30.15 | 39.48 | 4.66 |
| Sum 6 - 10 | 15.13 | 17.67 | 8.70 | 22.07 | 32.21 | 18.11 | 25.79 | 10.04 | 21.84 | 14.32 | 18.59 | 2.27 |

**Table 14 - Active Application**

| | Test Subject A | | Test Subject B | | Test Subject C | | Test Subject D | | Test Subject E | | | |
|---|---|---|---|---|---|---|---|---|---|---|---|---|
| | Site 1 | Site 2 | Site 1 | Site 2 | Site 1 | Site 2 | Site 1 | Site 2 | Site 1 | Site 2 | Avg | SEM |
| 1 | 4.39 | 4.57 | 2.35 | 3.27 | 4.14 | 4.35 | 6.97 | 1.98 | 5.72 | 6.53 | 4.43 | 0.52 |
| 2 | 5.60 | 5.81 | 4.15 | 4.81 | 7.59 | 6.44 | 11.60 | 2.73 | 8.60 | 3.72 | 6.10 | 0.83 |
| 3 | 6.37 | 11.49 | 5.16 | 6.52 | 10.32 | 7.03 | 15.59 | 2.97 | 11.66 | 6.57 | 8.37 | 1.19 |
| 4 | 7.51 | 14.02 | 5.37 | 9.34 | 10.30 | 7.12 | 11.19 | 3.17 | 8.49 | 5.95 | 8.25 | 0.99 |
| 5 | 6.56 | 11.52 | 5.30 | 9.40 | 10.29 | 6.51 | 10.99 | 4.30 | 6.66 | 5.25 | 7.68 | 0.83 |
| 6 | 5.67 | 10.12 | 3.13 | 9.83 | 10.08 | 5.08 | 9.27 | 4.51 | 7.50 | 5.01 | 7.02 | 0.84 |
| 7 | 5.62 | 7.18 | 1.82 | 6.87 | 7.45 | 5.62 | 6.84 | 3.44 | 7.24 | 3.15 | 5.52 | 0.64 |
| 8 | 4.98 | 5.52 | 2.56 | 8.76 | 6.86 | 3.85 | 3.18 | 2.23 | 4.23 | 4.14 | 4.63 | 0.63 |
| 9 | 3.73 | 4.42 | 2.37 | 6.79 | 7.40 | 4.11 | 8.72 | 2.35 | 8.10 | 3.10 | 5.11 | 0.76 |
| 10 | 3.12 | 2.90 | 2.36 | 8.13 | 6.48 | 3.20 | 7.82 | 2.79 | 4.20 | 3.76 | 4.48 | 0.69 |
| Sum 2 - 10 | 49.17 | 72.98 | 32.23 | 70.46 | 76.77 | 48.95 | 85.20 | 28.48 | 66.67 | 40.65 | 57.16 | 6.27 |
| Sum 6 - 10 | 23.12 | 30.14 | 12.25 | 40.38 | 38.27 | 21.85 | 35.82 | 15.32 | 31.26 | 19.16 | 26.76 | 3.11 |

Table 15 compares the results of active application versus passive application based on the additive amounts of niacinamide recovered from tape strips 2 to 10 and 6 to 10. The enhancement values shown in Table 15 are calculated by dividing the active value from Table 14 by the passive value from Table 13. The average shown in the last column of Table 15 is calculated by averaging the enhancement values of all the test sites. In instances where the passive value from Table 13 was zero, resulting in a divide-by-zero situation, the enhancement value is not included for purposes of the average. The p-value is calculated using a paired t-test. As shown in Table 15, active application of the composition delivered about 1.5x as much niacinamide into the skin compared to passive application. This suggests that the specific magnetic applicator used in this example can drive niacinamide deeper into the skin where it can provide an improved skin care benefit.

**Table 15 - Comparison of Active v. Passive Application**

| | Test Subject A | | Test Subject B | | Test Subject C | | Test Subject D | | Test Subject E | | Avg | P-value |
|---|---|---|---|---|---|---|---|---|---|---|---|---|
| | Site 1 | Site 2 | Site 1 | Site 2 | Site 1 | Site 2 | Site 1 | Site 2 | Site 1 | Site 2 | | |
| Enhancement value Strips 2 - 10 | 1.63 | 1.77 | 1.47 | 1.51 | 1.26 | 1.19 | 1.39 | 1.59 | 1.54 | 1.35 | 1.47 | 5.969 x10⁻⁵ |
| Enhancement value Strips 6 - 10 | 1.53 | 1.71 | 1.41 | 1.83 | 1.19 | 1.21 | 1.39 | 1.53 | 1.43 | 1.34 | 1.46 | 3.316x10⁻⁴ |

### Example 4 - Coefficient of Friction

### Coefficient of Friction method

This method provides a means to determine the coefficient of friction of material surfaces herein. Wet coefficient of friction refers to the coefficient of friction measured on a surface on which a skin care composition is present. Dry coefficient of friction refers to the coefficient of friction measured on a surface on which a skin care composition is not present.

Coefficient of friction is the ratio of the force of friction between two bodies and the force pressing them together. In the present method, the instrument used to determine the coefficient of friction is a Bruker® UMT-2 tribometer. However, an equivalent tribometer may be used, as desired. A purple nitrile glove material is used as one of the two materials in the test. The other material the test surface (e.g.., skin contacting surface of the applicator or cover). The purple nitrile glove material is placed over the probe of the tribometer. The test surface to be measured is placed in contact with the nitrile-covered probe of the instrument, and the force is measured according to the manufacturer's operating instructions for the instrument.

FIG. 7 illustrates the system 700 used to measure coefficient of friction in this example. As shown in FIG. 7, a probe 702 covered with purple nitrile glove material is contacted with the skin-contacting surface 720 of an applicator 710. In this example, the cover has been removed from the applicator 710 and the magnetic array provides the skin-contacting surface 720. The skin-contacting surface of the cover (not shown) was also measured. Both the applicator surface 720 and the cover were tested with and without a skin care composition (Olay® ProX Deep Wrinkle Cream ® brand skin care composition available from the Procter&Gamble Co., Ohio). For measuring wet coefficient of friction, 0.1 g of the skin care composition was spread over the test surface. The rate of the probe was set to 1 mm/sec with a force of 100 grams.

Each leg of the test was repeated three times. The coefficient of friction results are shown in Table 13 below.

**Table 13**

| Surface | Coefficient of Friction | | | |
|---|---|---|---|---|
| | 1 | 2 | 3 | Avg. |
| Applicator surface (dry) | 1.90 | 1.97 | 1.86 | 1.91 |
| Applicator surface (wet) | 0.45 | 0.62 | 0.45 | 0.50 |
| Cover surface (dry) | 0.77 | 0.96 | 1.09 | 0.94 |
| Cover surface (wet) | 0.06 | 0.06 | 0.06 | 0.06 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

All documents cited in the Detailed Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A cosmetic skin care product, comprising:
a. an applicator comprising a substrate with a magnetic array embedded therein, the magnetic array comprising a first layer of at least one dipolar pair of alternating magnetic poles with a pitch of between 1 mm and 3.5 mm and a first layer magnetic field strength of between 12 mT and 30 mT; and
b. a skin care composition comprising an effective amount of a vitamin B3 active and a dermatologically acceptable carrier.

2. The product of claim 1, wherein the first layer has a thickness of between 0.2 mm and 1 mm.

3. The product of claim 1 or 2, wherein the first layer has a pitch of 2.1 mm, a thickness of 0.6 mm and a magnetic field strength of 23 mT.

4. The product of any preceding claim, wherein the magnetic substrate comprises a skin facing side and a distal side opposed thereto, and a magnetic return provided at the distal side.

5. The product of any preceding claim, wherein the substrate includes a ferromagnetic material selected from iron, iron containing materials, cobalt, cobalt containing materials, strontium, strontium containing materials, barium, barium containing materials, nickel, nickel containing materials, alloys and oxides of these, and combinations thereof.

6. The product of any preceding claim, wherein the magnetic array further comprises a second layer of at least one dipolar pair of alternating magnetic poles offset from the first layer at an angle of between 1° to 179°, the second layer of magnetic poles having a second layer pitch of between 1mm to 3.5 mm, and a second layer magnetic field strength of between 8 mT and 24 mT, wherein the second layer magnetic field strength is less than or equal to the first layer magnetic field strength.

7. The product of claim 6, wherein the overall magnetic field strength of the first and second layers is between 13 mT and 32 mT.

8. The product of claim 6 or 7, wherein the second layer pitch is equal to or less than the first layer pitch.

9. The product of any of claims 6 to 8, wherein the first layer and second layer of poles are magnetized on a single substrate having a thickness equivalent to the first layer thickness.

10. The product of any of claims 6 to 9, wherein the first layer and second layer are magnetized on separate substrates, each having a skin facing side and a distal side opposed thereto, and a magnetic return provided at the distal side, wherein the skin facing side of the first layer is arranged adjacent to and parallel with the distal side of the second layer.

11. The product of any of claims 6 to 10, wherein the magnetic array further comprises:
a. a first layer thickness of 0.6 mm, a first layer pitch of 2.1 mm;
b. a second layer thickness of 0.2 mm, a second layer pitch of 1.49 mm; and
c. wherein the overall magnetic field strength of the magnetic array is 27 mT.

12. A non-therapeutic method of cosmetically regulating a skin condition, comprising:
a. applying a skin care composition to a target portion of skin, the skin care composition including a vitamin B3 active and a dermatologically acceptable carrier;
b. positioning an applicator that includes the magnetic array of any of the preceding claims above the target portion of skin; and
c. moving the applicator such that the vitamin B3 compound in proximity to the applicator will be subject to alternating polarities of magnetic flux in response to said movement.

## Patentansprüche

1. Kosmetisches Hautpflegeprodukt, umfassend:
a. einen Applikator, der ein Substrat mit einer darin eingebetteten Magnetbaugruppe umfasst, wobei die Magnetbaugruppe eine erste Schicht aus mindestens einem dipolaren Paar von magnetischen Wechselpolen mit einem Abstand von zwischen 1 mm bis 3,5 mm und einer Magnetfeldstärke der ersten Schicht von zwischen 12 mT bis 30 mT aufweist; und
b. eine Hautpflegezusammensetzung, umfassend eine wirksame Menge eines Vitamin B3-Wirkstoffs und eines dermatologisch akzeptablen Trägers.

2. Produkt nach Anspruch 1, wobei die erste Schicht eine Dicke von zwischen 0,2 mm und 1 mm aufweist.

3. Produkt nach Anspruch 1 oder 2, wobei die erste Schicht einen Abstand von 2,1 mm, eine Dicke von 0,6 mm und eine Magnetfeldstärke von 23 mT aufweist.

4. Produkt nach einem der vorstehenden Ansprüche, wobei das magnetische Substrat eine hautseitige Seite und eine dazu gegenüberliegende distale Seite und einen magnetischen Rücksprung umfasst, der an der distalen Seite vorgesehen ist.

5. Produkt nach einem der vorstehenden Ansprüche, wobei das ferromagnetische Material ausgewählt ist aus Eisen, eisenhaltigen Materialien, Kobalt, kobalthaltigen Materialien, Strontium, strontiumhaltigen Materialien, Barium, bariumhaltigen Materialien, Nickel, nickelhaltigen Materialien, Legierungen und Oxiden dieser, und Kombinationen davon.

6. Produkt nach einem der vorstehenden Ansprüche, wobei die Magnetbaugruppe ferner eine zweite Schicht aus mindestens einem dipolaren Paar von magnetischen Wechselpolen umfasst, die von der ersten Schicht in einem Winkel von zwischen 1° bis 179° versetzt sind, wobei die zweite Schicht aus magnetischen Polen einen Abstand einer zweiten Schicht von zwischen 1 mm bis 3,5 mm und eine magnetische Feldstärke der zweiten Schicht von zwischen 8 mT bis 24 mT aufweist, wobei die magnetische Feldstärke der zweiten Schicht kleiner als oder gleich der magnetischen Feldstärke der ersten Schicht ist.

7. Produkt nach Anspruch 6, wobei die Gesamtmagnetfeldstärke der ersten und der zweiten Schicht zwischen 13 mT bis 32 mT liegt.

8. Produkt nach Anspruch 6 oder 7, wobei der Abstand der zweiten Schicht gleich oder kleiner als der Abstand der ersten Schicht ist.

9. Produkt nach einem der Ansprüche 6 bis 8, wobei die erste Schicht und die zweite Schicht von Polen auf einem einzigen Substrat mit einer Dicke magnetisiert sind, die der Dicke der ersten Schicht entspricht.

10. Produkt nach einem der Ansprüche 6 bis 9, wobei die erste Schicht und die zweite Schicht auf getrennten Substraten magnetisiert sind, die jeweils eine hautseitige Seite und eine ihr gegenüberliegende distale Seite aufweisen, und eine an der distalen Seite vorgesehene magnetische Rückführung, wobei die hautseitige Seite der ersten Schicht benachbart zu und parallel zu der distalen Seite der zweiten Schicht angeordnet ist.

11. Produkt nach einem der Ansprüche 6 bis 10, wobei die Magnetbaugruppe ferner Folgendes umfasst:
a. eine Dicke der ersten Schicht von 0,6 mm, einen Abstand der ersten Schicht von 2,1 mm;
b. eine Dicke der zweiten Schicht von 0,2 mm, einen Abstand der zweiten Schicht von 1,49 mm; und
c. wobei die Gesamtmagnetfeldstärke der Magnetbaugruppe 27 mT beträgt.

12. Nichttherapeutisches Verfahren zur kosmetischen Regulierung einer Hauterkrankung, das Folgendes umfasst:
a. Auftragen einer Hautpflegezusammensetzung auf einen Zielabschnitt der Haut, wobei die Hautpflegezusammensetzung einen Vitamin B3-Wirkstoff und einen dermatologisch akzeptablen Träger einschließt;
b. Positionieren eines Applikators, der die Magnetbaugruppe nach einem der vorstehenden Ansprüche einschließt, über dem Zielabschnitt der Haut; und
c. Bewegen des Applikators derart, dass die Vitamin B3-Verbindung in Nähe des Applikators wechselnden Polaritäten des magnetischen Flusses als Reaktion auf die Bewegung ausgesetzt ist.

## Revendications

1. Produit cosmétique de soin de la peau, comprenant :
a. un applicateur comprenant un substrat avec un réseau magnétique intégré en son sein, le réseau magnétique comprenant une première couche d'au moins une paire dipolaire de pôles magnétiques alternés avec un pas compris entre 1 mm et 3,5 mm et une intensité de champ magnétique de première couche comprise entre 12 mT et 30 mT ; et
b. une composition de soin de la peau comprenant une quantité efficace d'un principe actif à la vitamine B3 et un véhicule acceptable du point de vue dermatologique.

2. Produit selon la revendication 1, dans lequel la première couche a une épaisseur comprise entre 0,2 mm et 1 mm.

3. Produit selon la revendication 1 ou 2, dans lequel la première couche a un pas de 2,1 mm, une épaisseur de 0,6 mm et une intensité de champ magnétique de 23 mT.

4. Produit selon une quelconque revendication précédente, dans lequel le substrat magnétique comprend un côté faisant face à la peau et un côté distal opposé à celui-ci, et un retour magnétique fourni au niveau du côté distal.

5. Produit selon une quelconque revendication précédente, dans lequel le substrat inclut un matériau ferromagnétique choisi parmi le fer, des matériaux contenant du fer, le cobalt, des matériaux contenant du cobalt, le strontium, des matériaux contenant du strontium, le baryum, des matériaux contenant du baryum, le nickel, des matériaux contenant du nickel, des alliages et oxydes de ceux-ci et leurs combinaisons.

6. Produit selon une quelconque revendication précédente, dans lequel le réseau magnétique comprend en outre une deuxième couche d'au moins une paire dipolaire de pôles magnétiques alternés, décalée de la première couche selon un angle compris entre 1° et 179°, la deuxième couche de pôles magnétiques ayant un pas de deuxième couche compris entre 1 mm et 3,5 mm, et une intensité de champ magnétique de deuxième couche comprise entre 8 mT et 24 mT, dans lequel l'intensité de champ magnétique de deuxième couche est inférieure ou égale à l'intensité de champ magnétique de première couche.

7. Produit selon la revendication 6, dans lequel l'intensité de champ magnétique globale des première et deuxième couches est comprise entre 13 mT et 32 mT.

8. Produit selon la revendication 6 ou 7, dans lequel le pas de deuxième couche est égal ou inférieur au pas de première couche.

9. Produit selon l'une quelconque des revendications 6 à 8, dans lequel la première couche et la deuxième couche de pôles sont magnétisées sur un substrat unique ayant une épaisseur équivalente à l'épaisseur de première couche.

10. Produit selon l'une quelconque des revendications 6 à 9, dans lequel la première couche et la deuxième couche sont magnétisées sur des substrats indépendants, ayant chacun un côté faisant face à la peau et un côté distal opposé à celui-ci, et un retour magnétique fourni au niveau du côté distal, dans lequel le côté faisant face à la peau de la première couche est agencé adjacent et parallèle au côté distal de la deuxième couche.

11. Produit selon l'une quelconque des revendications 6 à 10, dans lequel le réseau magnétique comprend en outre :
a. une épaisseur de première couche de 0,6 mm, un pas de première couche de 2,1 mm ;
b. une épaisseur de deuxième couche de 0,2 mm, un pas de deuxième couche de 1,49 mm ; et
c. dans lequel l'intensité de champ magnétique globale du réseau magnétique est de 27 mT.

12. Procédé non thérapeutique de régulation cosmétique d'un état de la peau, comprenant :
a. l'application d'une composition de soin de la peau à une partie cible de peau, la composition de soin de la peau incluant un principe actif à la vitamine B3 et un véhicule acceptable du point de vue dermatologique ;
b. le positionnement d'un applicateur qui inclut le réseau magnétique selon l'une quelconque des revendications précédentes au-dessus de la partie cible de peau ; et
c. le déplacement de l'applicateur de telle sorte que le composé de vitamine B3 à proximité de l'applicateur sera soumis à des polarités alternées de flux magnétique en réponse audit mouvement.
